# EUROPÄISCHE PATENTSCHRIFT

(11) **EP 2 448 677 B1**
(45) Veröffentlichungstag und Bekanntmachung des Hinweises auf die Patenterteilung: **13.04.2016**
(21) Anmeldenummer: 10727004.3
(22) Anmeldetag: 02.07.2010
(51) Int. Cl.: C12Q 1/00, G01N 33/543, B01L 3/00

(54) **POINT OF CARE TESTSYSTEM UND VERFAHREN ZUM AUFBRINGEN EINER PROBE**
POINT OF CARE TESTSYSTEM AND PROCESS FOR APPLYING A SAMPLE
DISPOSITIF D`ANALYSE HORS LABORATOIRE ET PROCÉDÉS DE FOURNIR UN ÉCHANTILLON

(30) Priorität: 02.07.2009 DE 102009033008
(43) Veröffentlichungstag der Anmeldung: 09.05.2012
(73) Patentinhaber: DST Diagnostische Systeme & Technologien GmbH, 19059 Schwerin (DE)
(72) Erfinder: VON OLLESCHIK-ELBHEIM, Lars, 48151 Münster (DE); HÜNKEN, Mark, 19061 Schwerin (DE); DANGERS, Marc, 19059 Schwerin (DE)
(74) Vertreter: Simandi, Claus
(86) Internationale Anmeldenummer: PCT/EP2010/059508
(87) Internationale Veröffentlichungsnummer: WO 2011/000959

(56) Entgegenhaltungen:
- EP-A2- 0 791 829
- WO-A1-92/07655
- US-A1- 2004 053 352
- US-B1- 6 696 240

## Beschreibung

Die vorliegende Erfindung betrifft ein Testsystem oder Assaysystem (Nachweissystem)und Testverfahren vorzugsweise im Gebrauch für den Point-of-Care (PoC) Bereich.

In der Forschung, Diagnostik, oder einer Vielzahl weiterer Anwendungsfelder stellen analytische Labortests, die zur qualitativen oder/und quantitativen Bestimmung von Molekülen, Analyten bzw. deren Aktivität oder Zusammensetzung dienen, die Basis für weitreichende Aussagen bis hin zur Entwicklung neuer Verfahren oder Vorrichtungen dar. Die Basis sind die allgemein bekannten Methoden der DNA/RNA Analytik bzw. der Proteinanalytik. Ein weiteres Beispiel sind die Vielzahl von analytischen Verfahren und Methoden, die eingesetzt werden, um die Antikörperreaktionen, sogenannte Immunreaktionen, welche für die Bestimmung von (Bio)Markern und vielen weiteren Substanzen/Analyten eingesetzt werden.

Es sind Schnelltestverfahren bekannt, wie der Lateral-Flow-Test (LFT), Flow-Through-Test (FTT), Agglutinations-Test (AT) oder Solid-Phase-Test (SPT). Alle diese Verfahren dienen zum schnellen Nachweis von Analyten ohne die Verwendung von Geräten und sind zur visuellen Auswertung geeignet.

Ein robustes Assayprinzip ist bekannt, wie im Stand der Technik zur in-vitro Diagnostik als Immunoassay (IA), insbesondere als EIA, oder "binding assay" ("sandwich") beschrieben (Siehe z.B. EP0171150B1, EP 0063810B1). Ferner ist z.B. der fluorometrische-Nachweis von IgE im Rahmen eines Bindungsassays aus EP0119613 B1 bekannt. Darüber hinaus, sei auf das Schrifttum von Roger P. Ekins (z.B. WO 8401031 u.v.a.) verwiesen.
Ferner ist ein membrangestützer Bindungsassay, insbesondere von IgE aus Blut an Allergenen seit Ende der 1980-iger Jahre bekannt. Z.B. CHEMICAL ABSTRACTS, vol. 101, no. 25, 17th December 1984, page 578, abstract no. 228190b, Columbus, Ohio, US; B.J. WALSH et al.: "Allergen discs prepared from nitrocellulose: detection of IgE binding to soluble and insoluble allergens", & J. IMMUNOL. METHODS 1984, 73(1), 139-45.

Für den PoC Bereich ist beispielsweise der käuflich erhältliche Fast-Check-PoC der Anmelderin beschrieben (siehe EP1718970), der auf Basis eines membrangestützen Bindungsassays zum Allergienachweis aus Vollblut eingesetzt werden kann.

Es besteht jedoch ein hohes Bedürfnis ein Nachweissystem eines PoC Testsystems zu verbessern.

Ein besonderes Problem ist die gleichzeitige bzw. simultane Probenbenetzung der einzelnen Nachweisplätze. Dies ist zur Reproduzierbarkeit und Erzielung eines qualitativ hochwertigen Ergebnisses erforderlich.

Ein ähnlich bedeutendes Problem ist die Benetzung der Nachweisplätze mit einer jeweils gleichen Menge Probenflüssigkeit.

Im Stand der Technik sind Röhrensysteme, wie z.B. aus der WO2002072264 für Immunoassays bekannt, die eine kontrollierte und gezielte Probenzuführung auf einer Membran erlauben sollen.

Allerdings ist nachteilig, dass die bekannten Kanal- oder Röhrensysteme kein gleichförmiges Zubringen oder Homogenität der Einzelproben aus einer Gesamtprobe gewährleisten.

Daher ist es Aufgabe der vorliegenden Erfindung für ein PoC-Testsystem eine verbesserte Probenzuführung bereitzustellen, die ein verbessertes Ergebnis erzielt.

Überraschender Weise wird diese Aufgabe durch den Anspruch 1 gelöst. Erfindungsgemäß ist ein Testverfahren vorgesehen, wobei
a. mindestens ein Testreagenz auf einer ersten Oberfläche fixiert ist, und rückseitig eine zweite Oberfläche ausgebildet ist,
b. mindestens eine Probenflüssigkeit in einem Probenkanal geführt wird, wobei der Probenkanal in eine Mündungsfläche mündet, die der zweiten Oberfläche gegenüberliegt, wobei die zweite Oberfläche mindestens in einem Bereich benetzbar ist, der der Mündung gegenüberliegt,
c. und die Probenflüssigkeit durch Druckbeaufschlagung des Probenkanals aus seiner Mündungsöffnung austritt und einen an der Mündungsfläche haftenden Tropfen bildet,
d. wobei ein Kontakt zwischen Tropfen und einem Benetzungspunkt auf der zweiten Oberfläche erzeugt wird, indem der Tropfen durch die Druckbeaufschlagung des Probenkanals so vergrößert wird, dass der Tropfen den Benetzungspunkt berührt,
e. die Probenflüssigkeit durch die genannte Berührung des Benetzungspunkts direkt oder durch Kapillarwirkung der ersten Oberfläche und/oder Druckbeaufschlagung des Probenkanals von der zweiten Oberfläche in Richtung der ersten Oberfläche eindringt oder bis zur ersten Oberfläche durchtritt und mit dem Testreagenz reagiert,
f. der Probenkanal derart gestaltet ist, dass er sich endständig zur Mündungsfläche hin aufweitet, und dass ein Tropfen die Aufweitung zumindest teilweise benetzt.

Weitere vorteilhafte Durchführungsarten und Ausführungsformen gehen aus den Unteransprüchen hervor.

Ein Testreagenz kann beispielsweise Substanzen, wie Peptide, Proteine, Enzyme oder deren Substrate, Liganden oder Rezeptoren, Antikörper oder Antigene, DNA, RNA, PNA enthalten, aber auch Moleküle nicht biochemischen Ursprungs, wie beispielsweise Jod. Die Testreagenzien können Kombinationen dieser Molekülsorten enthalten oder deren Fragmente, Konjugate, modifizierte, etwa glykolisierte oder phosphorylierte Formen. Lipide und Zucker sind ebenfalls erfindunggemäß umfasst. Ferner können Naturstoffe und natürliche Extrakte in Testreagenzien vorkommen, wie dies bei Allergietests oder Nahrungsmittelunverträglichkeittests häufig der Fall ist.

Die Mündungsfläche und / oder die erste und / oder zweite Oberfläche eines Nachweisträgers kann beispielsweise aus Glas, Kunststoff, insbesondere transparentem Kunststoff bestehen. Bevorzugt sind Polycarbonate wie beispielsweise Macrolon, polyzyklische Olefine wie beispielsweise Topaz, Polypropylen, PMMA oder Polyimide. Die Fläche kann weiterhin beschichtet sein, beispielsweise mit Proteinen, insbesondere Antikörpern, Zuckern wie zum Beispiel Dextranen, oder aber mit Metallen, Glas, oder Kunststoffen, oder Carbonderivaten wie beispielsweise Carbon Nanotubes.

Ein oder mehrere Testreagenzien sind auf der ersten Oberfläche fixiert. Erfindungsgemäß können die Testreagenzien in einer Schicht unterhalb der ersten Oberfläche fixiert sein. Die Testreagenzien können zum Beispiel an der Oberfläche eingetrocknet sein, entweder allein oder in einer stabilisierenden Matrix, oder als Lyophylisat aufgetragen werden und lösen sich bei Benetzung von der Oberfläche. Die Fixierung kann weiterhin durch Ausnutzung nicht kovalenter Wechselwirkungen erfolgen, wie sie beispielsweise zwischen Antikörper und Protein A bestehen, zwischen Biotin und Streptavidin, zwischen Hexa-Histidin und Nickel-NTA oder bei der Basenpaarung von DNA. Ferner kann ein Element der Testreagenzien kovalent an die erste Oberfläche gebunden sein. In einer bevorzugten Ausführungsform bleibt das Testreagenz bei Benetzung im Wesentlichen an der ersten Oberfläche fixiert, insbesondere zu mehr als 50%.

Eine Probenflüssigkeit kann eine beliebige Substanz oder Substanzgemisch, ggfs. samt Lösungsmittel, beliebiger Herkunft sein, insbesondere von einer Pflanze oder einem Tier stammen, insbesondere von einem Säugetier, insbesondere von einem Menschen. Die Probenflüssigkeit kann beispielsweise nicht abschließend Vollblut, Halbblut, Serum, Speichel, Tränenflüssigkeit, Urin, Sekret, Hirnflüssigkeit oder prozessierte Formen solcher Flüssigkeiten, wie etwa EDTA - stabilisiertes Blut enthalten.

Der Probenkanal kann ein Mittel zur Druckbeaufschlagung aufweisen. Das kann in einer bevorzugten Ausführungsform eine Befüllöffnung oder ein Befüllstutzen sein, in den zum Beispiel eine Spritze gesteckt wird, mit der ein Benutzer manuell Druck ausüben kann. Weitere Ausführungsformen sehen Blister oder Pumpkammern vor, oder Anschlüsse für externe Pumpen. Ferner kann auch eine Heizvorrichtung vorgesehen sein, mit der die Flüssigkeit ausgedehnt oder zur Verdampfung gebracht wird, wodurch Druck aufgebaut wird.

Die Mündungsfläche kann im Bereich der Mündung des Probenkanals benetzbar beschaffen sein, so dass der bei Druckbeaufschlagung entstehende Tropfen haftet und sich nicht unkontrolliert löst. Diese erfindungsgemäße Eigenschaft erfüllen nicht abschließend Polycarbonate wie beispielsweise Macrolon und Polyolefine wie Topaz in unbehandelter Form, oder nachbehandelt, sofern die Nachbehandlung wie etwa Plasmabehandlung die Benetzbarkeit nicht oder nur gering mindert. Dieser benetzbare Bereich kann von einem hydrophoben Bereich umgeben sein, mit dem die Tropfengröße beschränkt werden kann.

Die zweite Oberfläche ist in einem Bereich des Benetzungspunkts, der der Mündung gegenüberliegt, benetzbar beschaffen, so dass der Tropfen die zweite Oberfläche berührt. Insbesondere kann dieser Bereich aus Nitrozellulose bestehen.

Besonders bevorzugt ist das Verfahren, wenn die zweite Oberfläche an der Mündung des Probenkanals einen Abstand zur Mündungsfläche von 0,001 mm bis 15 mm, insbesondere von 0,01 mm bis 2 mm, insbesondere von 0,04 bis 0,4 mm aufweist.

Der Benetzungspunkt liegt auf der zweiten Oberfläche und ist der erste Punkt dieser Oberfläche, den der Tropfen bei seinem Wachstum auf der zweiten Oberfläche berührt. Besonders bevorzugt sind Ausführungsformen, bei denen der Benetzungspunkt vollständig reproduzierbar ist, wie sie durch die benannte Hydrophobisierung auf der Mündungsfläche, und/oder durch Hydrophobisierung um den Benetzungspunkt herum auf der zweiten Oberfläche, und / oder besonders bevorzugt durch eine Aufweitung um die endständige Öffnung des Probenkanals erzeugt werden kann.

In einer besonders bevorzugten Ausführungsform des Verfahrens ist der Probenkanal daher derart gestaltet, dass er sich endständig zur zweiten Oberfläche hin aufweitet, so dass der Tropfen die Oberfläche der Aufweitung zumindest teilweise oder ganz benetzt. Die Aufweitung reicht erfindungsgemäß von der Mündung des Probenkanals bis zum Mündungsrand der Aufweitung.

Erfindungsgemäß ist die Aufweitung im Bereich des Mündungsrandes benetzbar. Es kann bevorzugt sein, dass der Bereich des Mündungsrandes der Aufweitung hydrophob beschaffen ist.

Die Aufweitung bewirkt vorteilhaft, dass Kontaktwinkel und Tropfenoberfläche energetisch optimal sind, wenn der Tropfen sich in der Aufweitung bildet. Bei der Ausdehnung über die Grenzen der Aufweitung hinweg muss dagegen mehr Energie aufgebracht werden. Damit bewirkt die Aufweitung, dass der Tropfen eine vorteilhafte stabile und reproduzierbare Lage und Form einnimmt. Der Benetzungspunkt auf der zweiten Oberfläche und der Durchtrittspunkt der Probenflüssigkeit auf der ersten Oberfläche sind auf diese Weise vorteilhaft präzise festgelegt.

Besonders bevorzugt ist das Verfahren, wenn die Mündungsfläche an der Position am Mündungsrand der Aufweitung einen Abstand zur zweiten Oberfläche von 0,001 mm bis 15 mm, insbesondere von 0,01 mm bis 2 mm, insbesondere von 0,04 bis 0,4 mm aufweist.

In einer weiteren Ausführungsform kann die zweite Oberfläche an mindestens einem Mündungsrand einer Aufweitung abschließen oder zumindest teilweise aufliegen oder ein Zwischenraum ist von der zweiten Oberfläche zum Mündungsrand der Aufweitung ausgebildet. Der gebildete Raum stellt eine Kavität dar, der räumlich abgrenzbar ist (z.B. mittels einem Gehäuse, Seitenwände etc.).

Weiterhin sind die einzelnen Mündungsränder der Aufweitung vorzugsweise in einer einzigen Mündungsfläche ausgestaltet, die der ersten und zweiten Oberfläche gegenüber liegt und / oder der zweiten Oberfläche ganz oder teilweise anliegt.

Ein Zwischenraum kann daher unterschiedliche Volumensäulen von der zweiten Oberfläche oder ersten Oberfläche zu einem jeden Mündungsrand einer Ausweitung annehmen. Beispielsweise kann die zweite Oberfläche eine schiefe Ebene zu der Mündungsfläche aus der Gesamtheit von Probenkanälen/Ausweitungen aufweisen. Insbesondere kann die zweite Oberfläche auf der Ebenen der Mündungsränder der Aufweitungen partiell aufliegen, sei es an den Mündungsrändern der Aufweitungen oder neben den Aufweitungen.

Besonders bevorzugt ist das Verfahren, wenn die mündungsseitige Aufweitung mindestens eines der Probenkanäle konisch oder konkav, insbesondere halbkugelig, in Form eines Tellers oder einer Kugelkappe ausgebildet ist. Die Aufweitung kann auch beliebige, annähernd ovale oder im Wesentlichen konkave Formen annehmen. Die Aufweitung kann auch eine zylindrische Gestalt aufweisen, oder allgemein Kanten und Knicke aufweisen. Es kann bevorzugt sein, die Aufweitung trompetenförmig zu formen.

In einer bevorzugten Ausführungsform hat die Aufweitung an dem Mündungsrand einen Durchmesser zwischen 0,015 und 15 mm, insbesondere zwischen 0,1 und 6 mm, insbesondere zwischen 1 und 4 mm.

In einer bevorzugten Ausführungsform hat die Aufweitung eine Tiefe, d.h. einen Abstand von der Mündung des Probenkanals bis zum Mündungsrand der Aufweitung von 0,001 mm bis 2 mm, insbesondere zwischen 0,01 und 1 mm.

Des Weiteren kann der Probenkanal zusätzlich einen Mündungsstutzen aufweisen, sofern dieser die Benetzung der Aufweitung nicht einschränkt.

Die Aufweitung kann von einer radialsymmetrischen Form abweichen, beispielsweise elliptisch oder oval, oder unregelmäßigen Querschnitt aufweisen.

Bevorzugt ist zudem ein nahezu gleicher Abstand der zweiten Oberfläche zu den Mündungsrändern über einen großen Teil der ersten Oberfläche und über mehrere Benetzungspunkte hinweg. Weiterhin ist bevorzugt, dass die erste und / oder zweite Oberfläche parallel und / oder planar zur Ebenen der Mündungsränder bzw. Mündungsfläche aus der Gesamtheit von Probenkanälen/Ausweitungen verläuft.

Es kann fertigungstechnisch bedingt sein, dass dieser Abstand zwischen Mündungsfläche und zweiter Oberfläche nicht genau eingehalten werden kann oder nicht stabil ist, beispielweise wenn eine Membran als erste Oberfläche und zweite Oberfläche verwendet wird. Das erfindungsgemäße Verfahren weist den besonderen Vorteil auf, dass auch unter dieser Bedingung die Benetzung der Benetzungspunkte nahezu gleichförmig und gleichzeitig erfolgt.

Besonders bevorzugt ist eine Ausführungsform, bei der dem Test durch mindestens einen weiteren Kanal mindestens ein (Test-, Nachweis)Reagenz, insbesondere eine Waschlösung und / oder eine Stopplösung zugeführt wird.

Ein oder mehrere Kanäle können aus verschiedenen Richtungen zum Ort führen, an dem ein Testreagenz fixiert ist, und / oder zum Benetzungspunkt. Bevorzugt ist, den weiteren Kanal senkrecht zur Linie Probenkanal - Benetzungspunkt (zweite Oberfläche) - ggfs. Durchtrittspunkt (erste Oberfläche) anzuordnen. Insbesondere kann der weitere Kanal mehrere Messplätze seriell oder parallel mit einem Reagenz versorgen. Das Reagenz kann beispielsweise ein Stoppreagenz sein, das eine enzymatische Reaktion, beispielsweise bei einem ELISA, stoppt. Das Reagenz kann beispielsweise ein Substrat enthalten, das bei einem Lumineszenzverfahren (Firefly) die Lichtemission einleitet. Besonders bevorzugt ist eine Ausführungsform, bei der ein markierter Sekundärantikörper zugeführt wird. Des Weiteren ist bevorzugt, eine Waschlösung zuzuführen, beispielsweise um nicht bindende Antikörper vom Messplatz wegzuspülen. Es kann bevorzugt sein, mehrere (Test-)Reagenzien durch den gleichen Kanal nacheinander zuzuführen.

Weiterhin bevorzugt ist eine Ausführungsform, bei der die Mündungsfläche und die zweite Oberfläche starr miteinander verbunden sind, bei der insbesondere das Volumen zwischen Mündung des Probenkanals samt Aufweitung und zweiter Oberfläche ggfs. samt Zwischenraum abgeschlossen gegenüber der äußeren Umgebung ist und eine erste Kavität bildet, und bei der die in diesem Volumen befindliche Luft durch einen weiteren Kanal oder durch mindestens eine weitere Öffnung entweichen kann. Die erste Kavität kann unterschiedliche Formen annehmen, sofern sie gewährleistet, dass der Tropfen den Benetzungspunkt der ersten Oberfläche berühren kann. Besonders bevorzugt ist eine Ausführungsform, bei der die Kavität an mindestens einer Mündung eines Probenkanals einen Abstand von der Mündung zur zweiten Oberfläche von 0,001 mm bis 15 mm, insbesondere von 0,01 mm bis 2 mm, insbesondere von 0,04 bis 0,4 mm aufweist.

Diese Kavität kann weiterhin einen oben ausgeführten Zwischenraum umfassen.

Besonders bevorzugt ist eine Ausführungsform, bei der die erste und / oder zweite Oberfläche aus einer gelartigen, porösen, siebartigen, permeablen oder semipermeablen Membran, oder Dialysemembran, insbesondere einer beschichteten oder unbeschichteten Nitrozellulose-Membran besteht.

Erfindungsgemäß kann die erste und / oder zweite Oberfläche ganz oder teilweise mit dem Substratmaterial der Kavität verbunden sein. Sie kann auch als Beschichtung aufgebracht sein und durch bekannte mikrotechnische Verfahren in situ hergestellt sein, beispielweise durch drucken, bedampfen oder Sol-Gel Verfahren, und kann von Ihrem Substrat gelöst werden durch das Auflösen einer vorher aufgebrachten Opferschicht. Die erste und / oder zweite Oberfläche kann durch bekannte Verfahren strukturiert werden, beispielsweise durch Aufbringen einer schützenden Maske auf beizubehaltende Bereiche und nachfolgendes Ätzen oder Plasmabehandlung. Die erste und / oder zweite Oberfläche kann aus mehreren Schichten aufgebaut sein und in situ bearbeitet werden, beispielsweise können mit einem Laser Löcher in die erste und / oder zweite Oberfläche erzeugt worden sein, oder mindestens eine ihrer Schichten kann durch Plasmabehandlung oder mit Biomolekülen funktionalisiert sein.

Es kann bevorzugt sein, den Abstand zwischen erster und zweiter Oberfläche im Bereich von 0,001 bis 1 mm auszuführen. In einer bevorzugten Ausführungsform ist sie monomolekular.

In einer bevorzugten Ausführungsform wird eine Membran, zumindest teilweise außerhalb gefertigt und erst nach diesen Fertigungsschritten auf eine Oberfläche aufgebracht oder dazwischen positioniert. Die Membran kann beispielsweise auf zumindest einem Teil ihrer Fläche geklebt werden, durch statische Aufladung fixiert, oder mechanisch geklemmt sein, insbesondere zwischen erster und zweiter Oberfläche, oder durch Kombinationen verschiedener Verfahren befestigt sein.

Erfindungsgemäß wird die Membran vor der Positionierung und außerhalb mit mindestens einem Testreagenz versehen. Die Testreagenzien können durch eine Dispensiereinheit auf die Membran aufgebracht sein, durch Abstreifen von einem Gegenstand, insbesondere einer Röhre mit oder ohne Nachfülleinrichtung, durch Druckverfahren, beispielsweise Siebdruck, durch photochemische Fixierung oder mittels eines Sprays. Es können auch Linkermoleküle an der Membran verankert werden, an die die Moleküle des Testreagenz binden, ohne die Bindung der Analyten aus der Probenflüssigkeit wesentlich zu beeinflussen.

In einer anderen Ausführungsform wird zumindest ein Teil der Testreagenzien in situ aufgebracht. Dabei können beispielsweise die benannten Verfahren eingesetzt werden.

In einer besonders bevorzugten Ausführungsform ist die Membran Wasser aufsaugend, so dass sich die Probenflüssigkeit nach Benetzung des Benetzungspunkts mit dem Tropfen sich weiter auf oder in der Membran ausbreitet. Die Ausbreitung kann durch Kapillarkraft und / oder kontinuierlicher Druckbeaufschlagung der Probenflüssigkeit erfolgen. Insbesondere kann die Membran aus einem vliesartigen Material bestehen, und insbesondere aus Nitrozellulose.

Es kann bevorzugt sein, die Membran mit einem weiteren Material, beispielsweise Watte zu verbinden, das die Kapazität für die Kapillarität, d.h. die Saugleistung erhöht.

Es ist besonders bevorzugt, die Membran in Form von mindestens einem Streifen in der Kavität zu positionieren, insbesondere in einer zueinander parallelen Positionierung.

In einer besonders bevorzugten Ausführungsform werden die Membranen oder Streifen mit Testreagenzien gespottet, bedruckt, oder die Testreagenzien werden berührend aufgetragen.

In einer besonders bevorzugten Ausführungsform werden die Testreagenzien in Form von Streifen, Quadraten oder Rechtecken, Kreisen, Zahlen oder Buchstaben, oder Symbolen aufgebracht. Besonders bevorzugt ist weiterhin, andere schwarz-weisse, graue oder farbige Symbole vorab ohne Testreagenz aufzubringen, so dass beispielsweise aus einem vorab aufgebrachten "-" Zeichen ein "+"-zeichnen wird.

In einer besonders bevorzugten Ausführungsform ist das Testreagenz als Streifen auf der Membran aufgebracht, der von einer Kante der Membran bis zur anderen reicht. Besonders bevorzugt ist die Membran streifenförmig, und die Streifen Testreagenz reichen von einer Längskante des Membranstreifens bis zur gegenüberliegenden Längskante.

In einer bevorzugten Ausführungsform befindet sich auf der ersten Oberfläche eine zweite Kavität die unabhängig von der gebildeten Kavität an der zweiten Oberfläche ist, in die mindestens ein mikrofluidischer Kanal münden kann, der diese zweite Kavität mit einem Reservoir oder mit dem Außenraum verbindet.

Die zweite Kavität kann unterschiedlich ausgestaltet sein, ohne dass das erfindungsgemäße Verfahren beeinträchtigt wird. Insbesondere kann es bevorzugt sein, mikrofluidische Kanäle in der zweiten Kavität münden zu lassen, die mit einem Reservoir oder der äußeren Umgebung verbunden sind.

In einer besonders vorteilhaften Ausführungsform befindet sich kein Testreagenz am Durchtrittspunkt auf der ersten Oberfläche. Erfindungsgemäß liegt ein solcher Durchtrittspunkt auf der ersten Oberfläche dem ersten Benetzungspunkt auf der zweiten Oberfläche gegenüber. Sobald der Tropfen den Benetzungspunkt der zweiten Oberfläche berührt, breitet sich die Probenflüssigkeit durch Kapillarkraft und/oder Druck weiter auf der Membran aus, und erreicht mindestens einen Nachweisplatz mit Testreagenz, der auf einem genannten, abgedeckten Teilbereich der Membran liegen kann. Bei dieser Ausführungsform trägt die Membran auf der ersten Oberfläche nicht nur das Testreagenz, sondern hindert auch größere partikuläre Bestandteile der Probenflüssigkeit daran, das Testreagenz zu berühren.

Diese Ausführungsform erlaubt vorteilhaft, dass größere Bestandteile in einer Probenflüssigkeit, wie z.B. Erythrozyten einer Vollblutprobe, nicht mit den Testfeldern wechselwirken können.

Daher betrifft die Erfindung ebenfalls solche Ausführungsformen, in denen auf der ersten Oberfläche der Durchtrittspunkt von Testreagenzien frei ist.

In einer weiteren Ausführungsform sind mindestens zwei, insbesondere mindestens zehn Nachweisplätze an voneinander getrennten Stellen auf der ersten Oberfläche platziert. Ein Teil der Plätze kann auch für Kontrollbestimmungen vorgesehen sein. Bevorzugt ist, verschiedene Nachweise, insbesondere verschiedene Allergene oder Nahrungsmittelunverträglichkeitssubstanzen, auf verschiedenen Nachweisplätzen durchzuführen.

In einer bevorzugten Ausführungsform münden mindestens zwei Probenkanäle mit je einer Aufweitung in die Mündungsfläche, und mindestens zwei dieser Probenkanäle sind über mindestens eine Verzweigung mit mindestens einem gemeinsamen Reservoir oder einer gemeinsamen Befüllöffnung verbunden.

In einer besonders bevorzugten Ausführungsform weist mindestens ein Probenkanal endständig vor der Aufweitung eine Verengung oder Drossel auf, die den Fließwiderstand erhöht, der nach Hagen-Poiseuille durch Verringerung und/oder Veränderung der Form der Querschnittsfläche des Probenkanals erreicht werden kann. Bevorzugt ist eine Verringerung der Querschnittsfläche um mehr als 1%, insbesondere mehr als 10%, insbesondere von mehr als 30%. Besonders bevorzugt ist eine Änderung eines eckigen Querschnitts von 0,5 * 1 mm zu rundem Durchmesser von 0,5 mm. Der Querschnitt der Drossel kann von dem des Probenkanals abweichen und quadratische, runde, oder unregelmäßige Querschnitte aufweisen. Es kann bevorzugt sein, den Querschnitt über die Länge der Drossel in Form oder Größe zu variieren. In einer weiteren Ausführungsform verläuft die Drossel nicht gerade, sondern ist in ihrem Verlauf geknickt oder mäandrierend.

In einer weiteren bevorzugten Ausführungsform besteht die Verengung des jeweiligen Probenkanals auf einer Länge von 1 cm bis 0,5 mm.

In einer bevorzugten Ausführungsform erfolgt die Verengung in Form einer Stufe.

Es kann bevorzugt sein, das der Probenkanal senkrecht vom ersten Probenkanal abweicht, oder in einem Winkel von 40 bis 120 Grad bezüglich der Längsachse des ersten Probenkanals.

In einer bevorzugten Ausführungsform entstammen ein oder mehrere Probenkanäle aus einem ersten Probenkanal, wobei der Übergang vom ersten Probenkanal zu dem mindestens einen nachfolgenden Probenkanal eine Reduktion des Querschnitts aufweist, die auch die Funktion einer Drossel aufweist.

In einer besonders bevorzugten Ausführungsform entstammen ein oder mehrere, insbesondere vier Probenkanäle in gekreuzter Form aus einem ersten Probenkanal.

In einer bevorzugten Ausführungsform entstammen mindestens zwei, insbesondere alle Mündungen der Probenkanäle einem gemeinsamen Befüllstutzen.

In einer bevorzugten Ausführungsform sind die Querschnitte der Probenkanäle unterschiedlich und derart ausgelegt, dass unterschiedliche Laufwiderstände der Probenkanäle ausgeglichen werden. Damit wird erreicht, dass die unterschiedlichen Proben zu gleicher Zeit und in gleichen Mengen in die Aufweitung fließen, auch wenn die Längen der Kanäle oder die Anzahl der Knicke oder Biegungen pro Probenkanal unterschiedlich sind. Folglich ist die Größe von Tropfen, die in unterschiedlichen Aufweitungen entstehen, bei dieser Ausführungsform annähernd gleich.

In einer weiteren Ausführungsform kann die erfindungsgemäße Verengung in Form einer siebartigen Struktur erstellt werden, die im Wesentlichen quer zur Längsachse des Probenkanals positioniert ist. Die Durchmesser der einzelnen Sieböffnungen können unterschiedlich sein, ihre Anordnung unregelmäßig. Besonders bevorzugt ist eine quadratische oder hexagonale Anordnung der Sieböffnungen. In einer weiteren bevorzugten Ausführungsform werden die Sieböffnungen zum Rand hin größer, um das laminare Strömungsprofil im Probenkanal abzuflachen.

Es kann ferner bevorzugt sein, bei zwei oder mehr Probenkanälen die Durchmesser der Verengungen so zu variieren, dass verschiedene Probenkanäle ungeachtet ihrer unterschiedlichen Länge gleiche Strömungswiderstände aufweisen.

Diese Verengung oder Reduzierung des Durchmessers entlang des jeweiligen Probenkanals erlaubt eine optimale homogene Verteilung der Einzelproben aus einer Gesamtprobe, da eine Drosselung entlang des Strömungsgradienten erreicht wird und auf diese Weise eine gleichzeitige Tropfenbildung am Auslass des Probenkanals an der Membran zu einer optimalem Benetzung führt. Werden die Probenkanäle von einer gemeinsamen Befüllöffnung her mit Probenflüssigkeit gefüllt, füllen sich zunächst die Probenkanäle gleichzeitig. Bei Erreichen einer Verengung stoppt die Probenflüssigkeit zunächst und die anderen Probenkanäle befüllen sich weiter, bis bei allen die Verengung erreicht ist. Erst dann dringt die Probenflüssigkeit durch die Engstellen in die Aufweitungen vor und bildet dort jeweils einen Tropfen.

Hierbei ist die Benetzung mittels eines nach oben radial geöffneten Tellers besonders geeignet, da ein solcher Teller die Bildung eines Tropfens unterstützt. Der Tropfen dient der Überwindung des Abstandes zwischen dem Probenkanal und der Membran und ist abhängig von dem Durchmesser der Auslassmündung aus dem Kanal. D.h. die Tellerform führt zu einer starken Vergrößerung der Tropfenform und führt dazu, dass die Probe an der Benetzungsstelle in Kontakt mit dem Membranmaterial gebracht wird. Nachfolgend übernimmt die Membran eine saugende Rolle, bei der nach Kontaktaufnahme mit der Probe die Membran bis zur Absättigung passiv Probe aufnimmt.

In einer weiteren Ausführungsform kann ein jeder Probenkanal unabhängig voneinander gedrosselt sein. Eine solche Drosselung kann beispielsweise durch ein Ventil erfolgen, oder einfach mittels eines Stauraumes, wobei ein erster Probenkanal einen 40 - 120 Grad Winkel aufweist.

In einer weiteren bevorzugten Ausführungsform sind mindestens zwei Testreagenzien derart angeordnet, dass sie sternförmig mit gleichen oder unterschiedlichen Abständen oder Winkeln um einen Benetzungspunkt auf der ersten Oberfläche oder der Membran angelegt sind, so dass in gerader Verbindungslinie zwischen einem fixierten Testreagenz und dem Benetzungspunkt sich keine weitere fixierte Testreagenz befindet. Diese Anordnung vermeidet, dass die Probenflüssigkeit über verschiedene Nachweisplätze läuft und die Daten dieser Nachweisplätze sich beeinflussen, etwa durch kreuzreaktive Antikörper aus den Proben.

In einer bevorzugten Ausführungsform sind mehrere Membranen, insbesondere streifenförmige Membranen parallel angeordnet, und die zweite Kavität besteht aus einer oder mehreren parallelen Teilkavitäten. Insbesondere kann eine zweite Kavität so bemessen sein, dass sie sich über mehrere, bevorzugt alle Nachweisplätze eines Membranstreifens erstreckt. Bevorzugt ist ferner, dass die zweite Kavität sich ähnlich weit über einen Membranstreifen erstreckt. Ferner ist bevorzugt, dass der Membranstreifen erste und zweite Kavität vollständig voneinander trennt und keine Probenflüssigkeit von der ersten in die zweite Kavität gelangen kann, ohne durch die Membran hindurchzutreten.

In einer besonders bevorzugten Ausführungsform ist die zweite Kavität mit einem Befüllstutzen verbunden, durch den Testreagenzien oder Waschlösungen mit einer Spritze, Wassersäule, Pumpe oder anderen Vorrichtungen zur Druckerzeugung in die zweite Kavität gefüllt werden können. In einer besonders bevorzugten Ausführungsform ist diese Kavität mit einer Ausgangsöffnung oder mit einem Abfallbehälter verbunden, so dass Luft und überschüssige Flüssigkeit aus der zweiten Kavität entweichen kann. Bevorzugt sind zur zweiten Kavität weitere Kavitäten oder Teilkavitäten mit gemeinsamen Befüllstutzen oder Abfallbehältern miteinander verbunden.

In einer bevorzugten Ausführungsform ist über mindestens einem fixierten Testreagenz mindestens eine Linse angeordnet, so dass das menschliche Auge auch kleinere Nachweisplätze gut erkennen kann.

In einer bevorzugten Ausführungsform ist das Testreagenz lyophilisiert und / oder kovalent oder nicht kovalent an die erste Oberfläche oder Membran gekoppelt.

In einer bevorzugten Ausführungsform ist die Porengröße der ersten Oberfläche oder Membran so bemessen, dass Proteine die Poren passieren können, Erythrozyten oder weiße Blutkörperchen jedoch nicht. Insbesondere ist eine Porengröße von kleiner 0,8 um bevorzugt, ganz besonders kleiner 0,2 um.

In einer bevorzugten Ausführungsform beinhaltet das Testverfahren einen Bindungsassay. Dabei können beispielsweise Bindung zwischen Peptiden und/oder Proteinen oder DNA-, RNA-oder FNA-Hybridisierung, Antiköper-Antigen-Bindung, Aptamer-Target Bindung, Primär- Sekundärantikörper Wechselwirkung, Ligand-Rezeptor-Wechselwirkung oder Kombination davon verwendet werden. Besonders bevorzugt ist ein Bindungsassay, bei dem ein Antigen auf der ersten Oberfläche fixiert ist, die Probenflüssigkeit antigenspezifische IgE enthält, und ein Anti-IgE Antikörper hinzugegeben wird, der eine enzymatische, fluoreszente, lumineszente oder farbige Markierung trägt.

Die Erfindung betrifft weiterhin ein Testsystem bestehen aus einer ersten Oberfläche und zweiten Oberfläche, wobei
a. mindestens ein Testreagenz auf einer ersten Oberfläche fixiert ist, und rückseitig eine zweite Oberfläche ausgebildet ist,
b. wobei der Probenkanal in eine Mündungsfläche mündet, die der zweiten Oberfläche gegenüberliegt, wobei die zweite Oberfläche mindestens in einem Bereich benetzbar ist, der der Mündung gegenüberliegt,
c. wobei der Probenkanal endständig zur zweiten Oberfläche hin aufgeweitet ist, und dass ein Tropfen die Aufweitung zumindest teilweise benetzt,
wobei der Mündungsrand der Aufweitung einen Abstand zur zweiten Oberfläche von 0,001 mm bis 15 mm, insbesondere von 0,01 mm bis 2 mm, insbesondere 0,04 bis 0,4 mm aufweist und die erste und zweite Oberfläche, jeweils unabhängig voneinander eine Kavität ausbildet.

Das Testsystem kann durch vorstehende Merkmale entsprechend ausgebildet sein, auch wenn diese Merkmale im Bezug zum erfindungsgemäßen Verfahren stehen. Weitere Ausführungen sind in den Ansprüchen und Figuren dargelegt.

### Beispiele und Figuren:

Diese Beispiele dienen ausschließlich zur Erläuterung der Erfindung, ohne die Erfindung auf diese Beispiele zu beschränken.

### Beispiel 1:

A) Blutabnahme 100µl periphäres Blut, oder Serum oder heparinisiertes Vollblut
B) Mischen der Probe mit der Nachweislösung
C) Eingeben der Lösung in das Testgehäuse, die Nachweisfelder werden von ersten Oberfläche her homogen durchfeuchtet
D) 5 Minuten Inkubation
E) Über die Rückseite der nachweistragenden ersten Oberfläche wird 3x mit je 1 ml Waschflüssigkeit gewaschen, zwischen den Waschschritten sind je 1 ml Inkubationszeit.
F) Eingabe des Substrats über die Rückseite der Membran, 5 Minuten Inkubation
G) Abstoppen der Reaktion durch Eingabe von Stopplösung über die Rückseite der Membran
H) Visuelles Auslesen der Nachweisfelder

### Beschreibung der Figuren:

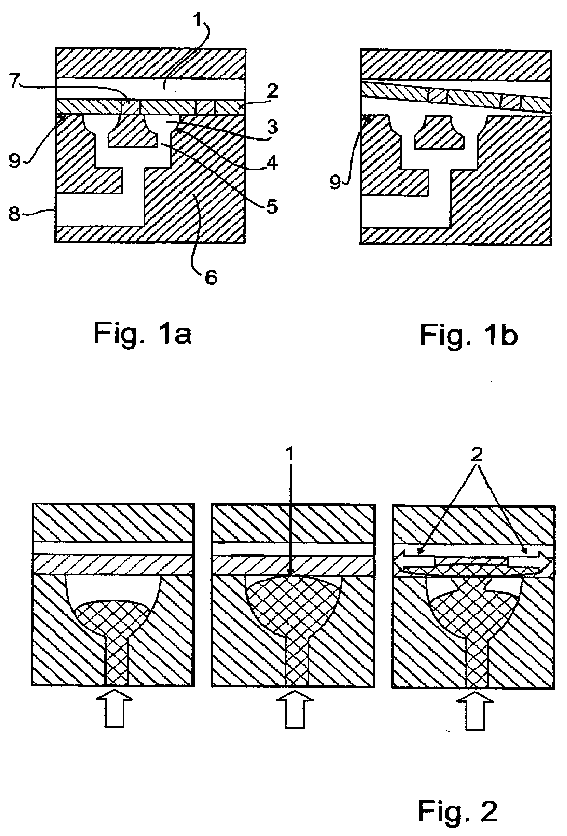

Figur 1 (a) zeigt eine mögliche Anordnung bestehend aus einer zweiten Kavität (1), Membran (2), Aufweitung (3), Oberfläche der Aufweitung(4), Verengung oder Drossel (5), Substrat (6), Nachweisplatz mit Testreagenz (7) und Probenkanal (8) und Mündungsfläche (9)
Figur 1 (b) zeigt, dass bei nicht an der Mündungsfläche (9) anliegender oder schräg liegender Membran die Anordnung eine nahezu gleichförmige Benetzung gewährleistet (Schräglage der Membran in der Zeichnung ist zur Verdeutlichung übertrieben).
Figur 2 zeigt den erfindungsgemäßen Benetzungsvorgang. Die Blockpfeile am unteren Bildrand geben die Ausbreitungsrichtung des Drucks in der Flüssigkeit an. (Links) Die Probenflüssigkeit tritt aus der Drossel aus und bildet einen Tropfen in der Aufweitung (Mitte) Der Tropfen wächst und berührt die Membran im Benetzungspunkt (1) (Rechts) Die Probenflüssigkeit breitet sich in der Membran aus, in Richtung (2) der Nachweisplätze.

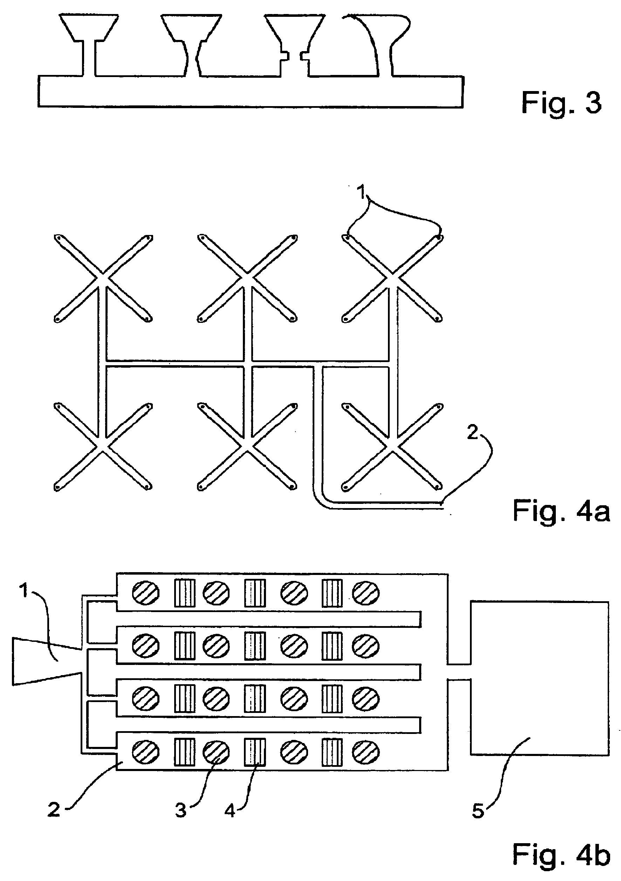

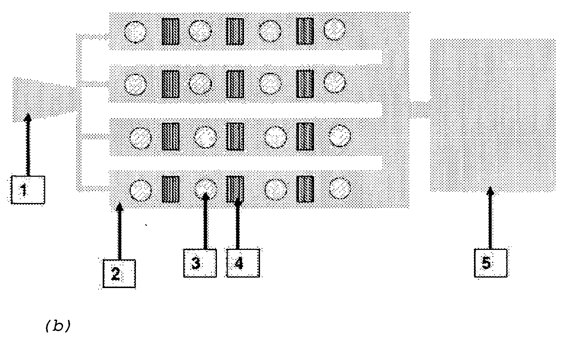

Figur 3 zeigt im Längsschnitt unterschiedliche Formen der Verengung oder Drossel. Die Aufweitungen sind jeweils als Trapeze dargestellt. Ganz rechts eine trompetenförmige Aufweitung.
Figur 4 (a) zeigt die Verteilung der Probenflüssigkeit in den Probenkanälen, die aus einem gemeinsamen Befüllstutzen (2) kreuzförmig zu der Drossel (1) und den sich anschließenden Aufweitungen geleitet wird.
Figur 4 (b)zeigt schematisch die Ebene der Zufuhr der Waschlösung über den gemeinsamen Befüllstutzen (1); mit den zweiten Kavitäten (2), in denen die Membranen liegen; den angedeuteten Aufweitungen (3) an der Unterseite der Membran (zweite Oberfläche)samt den Nachweisplätzen (4) eines Abfallbehälters (5).

## Patentansprüche

1. Testverfahren in einem Testsystem nach Anspruch 17, wobei:
a. mindestens ein Testreagenz auf einer ersten Oberfläche fixiert ist, und rückseitig eine zweite Oberfläche ausgebildet ist,
b. mindestens eine Probenflüssigkeit in einem Probenkanal (8, Fig 1a) geführt wird, wobei der Probenkanal (8, Fig 1a) in eine Mündungsfläche (9, Fig 1a) mündet, die der zweiten Oberfläche gegenüberliegt, wobei die zweite Oberfläche mindestens in einem Bereich benetzbar ist, der der Mündung gegenüberliegt,
c. und die Probenflüssigkeit durch Druckbeaufschlagung des Probenkanals (8, Fig 1a) aus seiner Mündungsöffnung austritt und einen an der Mündungsfläche (9, Fig 1a) haftenden Tropfen bildet,
d. wobei ein Kontakt zwischen Tropfen und einem Benetzungspunkt auf der zweiten Oberfläche erzeugt wird, indem der Tropfen durch die Druckbeaufschlagung des Probenkanals so vergrößert wird, dass der Tropfen den Benetzungspunkt berührt,
e. die Probenflüssigkeit durch die genannte Berührung des Benetzungspunkts direkt oder durch Kapillarwirkung der ersten Oberfläche und/oder Druckbeaufschlagung des Probenkanals von der zweiten Oberfläche in Richtung der ersten Oberfläche eindringt oder bis zur ersten Oberfläche durchtritt und mit dem Testreagenz reagiert,
**dadurch gekennzeichnet, dass**
f. der Probenkanal (8, Fig 1a) derart gestaltet ist, dass er sich endständig zur Mündungsfläche (9, Fig 1a) hin aufweitet, und dass ein Tropfen die Aufweitung (3, Fig 1a) zumindest teilweise benetzt.

2. Verfahren nach Anspruch 1, **dadurch gekennzeichnet, dass** die Mündungsfläche (9, Fig 1a) zumindest an der Position am Mündungsrand der Aufweitung (3, Fig 1a) einen Abstand zur zweiten Oberfläche von 0,001 mm bis 15 mm, insbesondere von 0,01 mm bis 2 mm, insbesondere 0,04 bis 0,4 mm aufweist.

3. Verfahren nach einem der vorhergehenden Ansprüche, **dadurch gekennzeichnet, dass** die zweite Oberfläche an mindestens einem Mündungsrand der Aufweitung (3, Fig 1a) abschließt oder zumindest teilweise auf der Aufweitung (3, Fig 1a) oder neben der Aufweitung (3, Fig 1a) aufliegt und / oder ein Zwischenraum von der zweiten Oberfläche zum Mündungsrand der Aufweitung (3, Fig 1a) ausgebildet ist.

4. Verfahren nach Anspruch 3, **dadurch gekennzeichnet, dass** eine Kavität (1, Fig 1a) ausgebildet wird.

5. Verfahren nach einem der vorhergehenden Ansprüche, **dadurch gekennzeichnet, dass** die mündungsseitige Aufweitung (3, Fig 1a) mindestens eines der Probenkanäle (8, Fig 1a) konisch oder konkav, insbesondere halbkugelig, in Form eines Tellers oder einer Kugelkappe ausgebildet ist.

6. Verfahren nach einem der vorhergehenden Ansprüche, **dadurch gekennzeichnet, dass** mindestens ein weiterer Kanal, ggfs. mindestens ein weiteres Reagenz, insbesondere eine Waschlösung oder eine Stopplösung zugeführt wird.

7. Verfahren nach einem der vorhergehenden Ansprüche, **dadurch gekennzeichnet, dass** die erste und / oder zweite Oberfläche aus einer gelartigen, porösen, siebartigen, permeablen oder semipermeablen Membran, Dialysemembran, insbesondere einer beschichteten oder unbeschichteten Nitrozellulose-Membran besteht.

8. Verfahren nach einem der vorhergehenden Ansprüche, **dadurch gekennzeichnet, dass** der Durchtrittspunkt auf der ersten Oberfläche frei von Testreagenz ist.

9. Verfahren nach einem der vorhergehenden Ansprüche, **dadurch gekennzeichnet, dass** die erste und zweite Oberfläche jeweils eine voneinander unabhängige Kavität (1, Fig 1a) ausbilden.

10. Verfahren nach einem der vorhergehenden Ansprüche, **dadurch gekennzeichnet, dass** mindestens ein Probenkanal endständig vor der Aufweitung (3, Fig 1a) eine Verengung oder Drossel (5, Fig 1a) aufweist.

11. Verfahren nach Anspruch 10, wobei mindestens eine Verengung eine Verringerung der Querschnittsfläche um mehr als 1%, insbesondere mehr als 10%, insbesondere von mehr als 30% aufweist.

12. Verfahren nach Anspruch 10 oder 11, **dadurch gekennzeichnet, dass** ein oder mehrere Probenkanäle aus einem ersten Probenkanal (8, Fig 1a) entstammen, wobei der erste Probenkanal eine Drosselung (5, Fig 1a) aufweist.

13. Verfahren nach einem der Ansprüche 10 bis 12, **dadurch gekennzeichnet, dass** zwei oder mehr Probenkanälen die Durchmesser der Verengungen variieren, so dass verschiedene Probenkanäle ungeachtet ihrer unterschiedlichen Länge gleiche Strömungswiderstände aufweisen.

14. Verfahren nach einem der vorhergehenden Ansprüche, **dadurch gekennzeichnet, dass** ein oder mehrere Probenkanäle in gekreuzter Form (1, Fig 4a) aus einem ersten Probenkanal (2, Fig 4a) entstammen.

15. Verfahren nach einem der vorhergehenden Ansprüche, dass mindestens zwei Testreagenzien derart angeordnet sind, dass sie sternförmig mit gleichen oder unterschiedlichen Abständen oder Winkeln um eine Mündung mindestens eines Probenkanals (8, Fig 1a) auf der ersten Oberfläche oder der Membran angelegt sind, so dass in gerader Verbindungslinie zwischen einem fixierten Testreagenz und der Mündung sich keine weitere fixierte Testreagenz befindet.

16. Verfahren nach einem der vorhergehenden Ansprüche, **dadurch gekennzeichnet, dass** das Testverfahren einen Bindungsassay beinhaltet.

17. Testsystem bestehend aus einer ersten Oberfläche und zweiten Oberfläche, **dadurch gekennzeichnet,**
**dass**
a. mindestens ein Testreagenz auf einer ersten Oberfläche fixiert ist, und rückseitig eine zweite Oberfläche ausgebildet ist,
b. wobei der Probenkanal (8, Fig 1a) in eine Mündungsfläche (9, Fig 1a) mündet, die der zweiten Oberfläche gegenüberliegt, wobei die zweite Oberfläche mindestens in einem Bereich benetzbar ist, der der Mündung gegenüberliegt,
**dadurch gekennzeichnet, dass**
c. der Probenkanal (8, Fig 1a) endständig zur zweiten Oberfläche hin aufgeweitet ist und dass ein Tropfen die Aufweitung (3, Fig 1a) zumindest teilweise benetzt,
wobei der Mündungsrand der Aufweitung (3, Fig 1a) einen Abstand zur zweiten Oberfläche von 0,001 mm bis 15 mm aufweist und die erste und zweite Oberfläche, jeweils unabhängig voneinander eine Kavität (1, Fig 1a) ausbildet.

18. Testsystem nach Anspruch 17, **dadurch gekennzeichnet, dass** mindestens ein Probenkanal (8, Fig 1a) endständig vor der Aufweitung (3, Fig 1a) eine Verengung oder Drossel (5, Fig 1a) aufweist.

19. Testsystem nach Anspruch 17 oder 18, **dadurch gekennzeichnet, dass** die mündungsseitige Aufweitung (3, Fig 1a) mindestens eines der Probenkanäle (8, Fig 1a) konisch oder konkav, insbesondere halbkugelig, in Form eines Tellers oder einer Kugelkappe ausgebildet ist.

20. Testsystem nach einem der vorhergehenden Ansprüche, **dadurch gekennzeichnet, dass** die Aufweitung (3, Fig 1a) am Mündungsrand einen Durchmesser zwischen 0,015 und 15 mm, insbesondere zwischen 0,1 und 6 mm, insbesondere zwischen 1 und 4 mm aufweist.

21. Testsystem nach einem der vorhergehenden Ansprüche, **dadurch gekennzeichnet, dass** die Aufweitung (3, Fig 1a) eine Tiefe, von 0,001 mm bis 2 mm, insbesondere zwischen 0,01 und 1 mm aufweist.

22. Testsystem nach einem der vorhergehenden Ansprüche, **dadurch gekennzeichnet, dass** der Durchtrittspunkt auf der ersten Oberfläche frei von Testreagenz ist.

23. Testsystem nach einem der vorhergehenden Ansprüche, **dadurch gekennzeichnet, dass** das Testsystem ein Bindungsassay ist, insbesondere geeignet für Blutproben.

## Claims

1. Test method in a test system according to claim 17, wherein
a. at least one test reagent is fixed on a first surface, and a second surface is formed on the reverse side thereof,
b. at least one sample fluid is conducted in a sample channel (8, figure 1a), wherein the sample channel (8, figure 1a) leads to a discharge area (9, figure 1a), which is disposed opposite the second surface, wherein the second surface is wettable at least in one region that is opposite the discharge area,
c. and the sample fluid exits the sample channel (8, figure 1a) through the discharge opening thereof as a result of applied pressure, and said fluid forms a drop that adheres to the discharge area opening (9, figure 1a),
d. wherein contact is produced between the drop and a wetting point on the second surface, in that, as a result of the pressure applied to the sample channel, the drop is enlarged such that the drop comes into contact with the wetting point,
e. the sample fluid, as a direct result of the stated contact with the wetting point, or as a result of the capillary action of the first surface and/or the pressure applied to the sample channel, penetrates from the second surface toward the first surface or travels up to the first surface and reacts with the test reagent,
**characterized in that**
f. the sample channel (8, figure 1a) is configured such that it is widened toward its end that faces the discharge area (9, figure 1a), and such that a drop at least partially wets the widened area (3, figure 1a).

2. The method according to claim 1, **characterized in that** the discharge area (9, figure 1a), at least at the position of the discharge opening edge of the widened area (3, figure 1a), is spaced from the second surface by 0.001 mm to 15 mm, especially by 0.01 mm to 2 mm, more particularly by 0.04 to 0.4 mm.

3. The method according to any one of the preceding claims, **characterized in that** the second surface ends on at least one discharge opening edge of the widened area (3, figure 1a) or can lie at least partially on the widened area (3, figure 1a) or beside the widened area (3, figure 1a), and / or an intermediate space is formed from the second surface up to the discharge opening edge of the widened area (3, figure 1a).

4. The method according to claim 3, **characterized in that** a cavity (1, figure 1a) is formed.

5. The method according to any one of the preceding claims, **characterized in that** the widened area (3, figure 1a) on the side of the discharge opening of at least one of the sample channels (8, figure 1a) is conical or concave, especially semispherical, in the form of a plate or a spherical segment.

6. The method according to any one of the preceding claims, **characterized in that** at least one additional reagent, especially a washing solution or a stop solution, is optionally supplied to at least one additional channel.

7. The method according to any one of the preceding claims, **characterized in that** the first and / or second surface consists of a gel-like, porous, screen-like, permeable or semipermeable membrane, or dialysis membrane, particularly a coated or uncoated nitrocellulose membrane.

8. The method according to any one of the preceding claims, **characterized in that** the point of passage on the first surface is free of test reagent.

9. The method according to any one of the preceding claims, **characterized in that** the first and second surfaces each form a cavity (1, figure 1a), independently of one another.

10. The method according to any one of the preceding claims, **characterized in that** at least one sample channel has a narrowed area or restrictor (5, figure 1a) near its end, upstream of the widened area (3, figure 1a).

11. The method according to claim 10, wherein at least one narrowed area has a reduction in cross-sectional area of more than 1%, especially more than 10%, more particularly more than 30%.

12. The method according to claim 10 or 11, **characterized in that** one or more sample channels originate from a first sample channel (8, figure 1a), wherein the first sample channel has a restrictor (5, figure 1a).

13. The method according to any one of claims 10 to 12, **characterized in that** two or more sample channels vary the diameter of the narrowed areas, and therefore different sample channels have the same flow resistances, irrespective of their different lengths.

14. The method according to any one of the preceding claims, **characterized in that** one or more sample channels in intersecting form (1, figure 4a) originate from a first sample channel (2, figure 4a).

15. The method according to any one of the preceding claims, **characterized in that** at least two test reagents are arranged such that they are disposed radially, at equal or unequal distances or angles, around a discharge opening of at least one sample channel (8, figure 1a) on the first surface or the membrane, so that no additional test reagent is located in a straight connecting line between a fixed test reagent and the discharge opening.

16. The method according to any one of the preceding claims, **characterized in that** the test method comprises a binding assay.

17. A test system consisting of a first surface and a second surface, **characterized in that**
a. at least one test reagent is fixed on a first surface, and a second surface is formed on the reverse side thereof,
b. wherein the sample channel (8, figure 1a) leads to a discharge area (9, figure 1a), which is disposed opposite the second surface, wherein the second surface is wettable at least in one area that is opposite the discharge area,
**characterized in that**
c. the sample channel (8, figure 1a) is widened at its end that faces the second surface, and **in that** a drop at least partially wets the widened area (3, figure 1a),
wherein the discharge opening edge of the widened area (3, figure 1a) is spaced from the second surface by 0.001 mm to 15 mm and the first and second surfaces each form a cavity (1, figure 1a), independently of one another.

18. The test system according to claim 17, **characterized in that** at least one sample channel (8, figure 1a) has a narrowed area or restrictor (5, figure 1a) near its end, upstream of the widened area (3, figure 1a).

19. The test system according to claim 17 or 18, **characterized in that** the widened area (3, figure 1a) of at least one of the sample channels (8, figure 1a), on the discharge opening side thereof, is conical or concave, particularly semispherical, in the form of a plate or a spherical segment.

20. The test system according to any one of the preceding claims, **characterized in that** the widened area (3, figure 1a) at the discharge opening edge has a diameter of between 0.015 and 15 mm, especially of between 0.1 and 6 mm, more particularly between 1 and 4 mm.

21. The test system according to any one of the preceding claims, **characterized in that** the widened area (3, figure 1a) has a depth of 0.001 mm to 2 mm, more particularly between 0.01 and 1 mm.

22. The test system according to any one of the preceding claims, **characterized in that** the point of passage on the first surface is free of test reagent.

23. The test system according to any one of the preceding claims, **characterized in that** the test system is a binding assay, particularly suitable for blood samples.

## Revendications

1. Procédé de test dans un système de test selon la revendication 17, dans lequel :
a. au moins un réactif de test est fixé sur une première surface, et une deuxième surface est formée sur la face arrière,
b. au moins un échantillon liquide est emmené dans un canal pour échantillon (8, Figure 1a), où le canal pour échantillon (8, Figure 1a) débouche dans une surface d'embouchure (9, Figure 1a), qui se situe en face de la deuxième surface, où la deuxième surface peut être mouillée sur au moins une zone qui se situe en face de l'embouchure,
c. et l'échantillon liquide sort de son ouverture d'embouchure par la mise sous pression du canal pour échantillon (8, Figure 1a) et forme une goutte qui adhère sur la surface d'embouchure (9, Figure 1a),
d. où un contact entre une goutte et un point de mouillage est créé sur la deuxième surface, par le fait que la goutte est agrandie par la mise sous pression du canal pour échantillon de telle sorte que la goutte touche le point de mouillage,
e. l'échantillon liquide pénètre en direction de la première surface ou traverse jusqu'à la première surface par l'intermédiaire dudit contact direct du point de mouillage ou par un effet capillaire de la première surface et/ou par la mise sous pression du canal pour échantillon par la deuxième surface et réagit avec le réactif de test,
**caractérisé en ce que**
f. le canal pour échantillon (8, Figure 1a) est conçu de telle façon qu'il s'évase à la fin vers la surface d'embouchure (9, Figure 1a) et qu'une goutte mouille au moins partiellement la partie évasée (3, Figure 1a).

2. Procédé selon la revendication 1, **caractérisé en ce que** la surface d'embouchure (9, Figure 1a) présente une distance par rapport à la deuxième surface de 0,001 mm à 15 mm, notamment de 0,01 mm à 2 mm, en particulier de 0,04 à 0,4 mm, au moins au niveau de la position du bord d'embouchure de la partie évasée (3, Figure 1a).

3. Procédé selon l'une des revendications précédentes, **caractérisé en ce que** la deuxième surface se termine au niveau d'au moins un bord d'embouchure de la partie évasée (3, Figure 1a) ou repose au moins partiellement sur la partie évasée (3, Figure 1a) ou à côté de la partie évasée (3, Figure 1a) et/ou qu'un espace intermédiaire est aménagé à partir de la deuxième surface jusqu'au bord d'embouchure de la partie évasée (3, Figure 1a).

4. Procédé selon la revendication 3, **caractérisé en ce qu'**une cavité (1, Figure 1a) est formée.

5. Procédé selon l'une des revendications précédentes, **caractérisé en ce que** la partie évasée (3, Figure 1a) du côté de l'embouchure d'au moins l'un des canaux pour échantillon (8, Figure 1a) est conçue conique ou concave, notamment sous la forme d'une demie sphère, sous la forme d'une assiette ou d'une calotte.

6. Procédé selon l'une des revendications précédentes, **caractérisé en ce qu'**au moins un autre canal, éventuellement, au moins un autre réactif, notamment une solution de lavage ou une solution d'arrêt, sont amenés.

7. Procédé selon l'une des revendications précédentes, **caractérisé en ce que** les première et/ou deuxième surfaces sont constituées d'une membrane de type gel, poreuse, faisant office de tamis, perméable ou semi perméable, d'une membrane de dialyse, notamment une membrane de nitrocellulose revêtue ou non revêtue.

8. Procédé selon l'une des revendications précédentes, **caractérisé en ce que** le point de passage sur la première surface est exempt de réactif de test.

9. Procédé selon l'une des revendications précédentes, **caractérisé en ce que** les première et deuxième surfaces forment chacune indépendamment l'une de l'autre une cavité (1, Figure 1a).

10. Procédé selon l'une des revendications précédentes, **caractérisé en ce qu'**au moins un canal pour échantillon présente un rétrécissement ou un étranglement (5, Figure 1a) à l'extrémité avant la partie évasée (3, Figure 1a).

11. Procédé selon la revendication 10, où au moins un rétrécissement présente une diminution de la surface transversale de plus de 1 %, en particulier de plus de 10 %, notamment de plus de 30 %.

12. Procédé selon les revendications 10 ou 11, **caractérisé en ce qu'**un ou plusieurs canaux pour échantillons sont issus d'un premier canal pour échantillon (8, Figure 1a), où le premier canal pour échantillon présente un étranglement (5, Figure 1a).

13. Procédé selon l'une des revendications 10 à 12, **caractérisé en ce que** deux ou plus de canaux pour échantillons font varier les diamètres des rétrécissements de sorte que des canaux pour échantillons différents présentent des résistances à l'écoulement identiques bien qu'ils soient de longueur différente.

14. Procédé selon l'une des revendications précédentes, **caractérisé en ce qu'**un ou plusieurs canaux pour échantillons en forme croix (1, Figure 4a) sont issus d'un premier canal pour échantillon (2, Figure 4a).

15. Procédé selon l'une des revendications précédentes, qu'au moins deux réactifs de test soient agencés de telle façon qu'ils soient disposés sous la forme d'une étoile avec des distances ou des angles identiques ou différents autour d'une embouchure d'au moins un canal pour échantillon (8, Figure 1a) sur la première surface ou sur la membrane de sorte qu'aucun autre réactif de test fixé ne se trouve en ligne droite de liaison entre un réactif de test fixé et l'embouchure.

16. Procédé selon l'une des revendications précédentes, **caractérisé en ce que** le procédé de test englobe un dosage de liaison.

17. Système de test constitué d'une première surface et d'une deuxième surface, **caractérisé en ce**
**que**
a. au moins un réactif de test est fixé sur une première surface, et une deuxième surface est formée sur le côté arrière,
b. où le canal pour échantillon (8, Figure 1a) débouche dans une surface d'embouchure (9, Figure 1a), qui est située en face de la deuxième surface, où la deuxième surface peut être mouillée au moins sur une partie qui se situe en face de l'embouchure,
**caractérisé en ce que**
c. le canal pour échantillon (8, Figure 1a) est élargi à l'extrémité vers la deuxième surface et qu'une goutte mouille au moins partiellement la partie évasée (3, Figure 1a),
où le bord d'embouchure de la partie évasée (3, Figure 1a) présente une distance par rapport à la deuxième surface de 0,001 mm à 15 mm et les première et deuxième surfaces forment chacune indépendamment l'une de l'autre une cavité (1, Figure 1a).

18. Système de test selon la revendication 17, **caractérisé en ce qu'**au moins un canal pour échantillon (8, Figure 1a) présente un rétrécissement ou un étranglement (5, Figure 1a) à l'extrémité avant la partie évasée (3, Figure 1a).

19. Système de test selon les revendications 17 ou 18, **caractérisé en ce que** la partie évasée (3, Figure 1a) d'au moins un des canaux pour échantillon (8, Figure 1a) est conçue conique ou concave, notamment en forme d'une demie sphère, sous la forme d'une assiette ou d'une calotte du côté de l'embouchure.

20. Système de test selon l'une des revendications précédentes, **caractérisé en ce que** la partie évasée (3, Figure 1a) sur le bord d'embouchure présente un diamètre entre 0,015 et 15 mm, en particulier entre 0,1 et 6 mm, notamment entre 1 et 4 mm.

21. Système de test selon l'une des revendications précédentes, **caractérisé en ce que** la partie évasée (3, Figure 1a) présente une profondeur de 0,001 mm à 2 mm, notamment entre 0,01 et 1 mm.

22. Système de test selon l'une des revendications précédentes, **caractérisé en ce que** le point de passage sur la première surface est exempt de réactif de test.

23. Système de test selon l'une des revendications précédentes, **caractérisé en ce que** le système de test est un dosage de liaison, en particulier approprié pour des échantillons de sang.
